# EUROPEAN PATENT SPECIFICATION

(11) **EP 1 660 025 B1**
(45) Date of publication and mention of the grant of the patent: **15.07.2009**
(21) Application number: 04772625.2
(22) Date of filing: 26.08.2004
(51) Int. Cl.: A61K 8/39, A61Q 19/10, A61K 8/64

(54) **COSMETIC COMPOSITION CONTAINING A POLYOXYETHYL COMBINATION AND A LIPOPEPTIDE**
KOSMETISCHE ZUSAMMENSETZUNG MIT EINER POLYOXYETHYL-KOMBINATION UND EINEM LIPOPEPTID
COMPOSITION COSMETIQUE COMPRENANT UN LIPOPEPTIDE ET UN ESTER D'ACIDE GRAS

(30) Priority: 28.08.2003 JP 2003303811
(43) Date of publication of application: 31.05.2006
(73) Proprietor: Showa Denko K.K., Tokyo 105-8518 (JP)
(72) Inventor: YONEDA, T., Corp. R & D Center, Showa Denko K.K., Chiba 267-0056 (JP)
(74) Representative: Strehl Schübel-Hopf & Partner
(86) International application number: PCT/JP2004/012669
(87) International publication number: WO 2005/020950

(56) References cited:
- WO-A-99/62482
- WO-A-20/05074881
- PATENT ABSTRACTS OF JAPAN vol. 2003, no. 09, 3 September 2003 (2003-09-03) & JP 2003 146827 A (KANEBO LTD), 21 May 2003 (2003-05-21)
- PATENT ABSTRACTS OF JAPAN vol. 2003, no. 10, 8 October 2003 (2003-10-08) & JP 2003 176211 A (SHOWA DENKO KK), 24 June 2003 (2003-06-24)
- PATENT ABSTRACTS OF JAPAN vol. 2003, no. 12, 5 December 2003 (2003-12-05) & JP 2003 277220 A (KANEBO LTD), 2 October 2003 (2003-10-02)

## Description

### TECHNICAL FIELD

The present invention relates to cosmetics. More specifically, the invention relates to a cosmetic comprising a lipopeptide compound derived from a microorganism, and a polyoxyethylene glyceryl ether fatty acid ester and/or a polyoxyethylene sorbit fatty acid ester, which ensures high skin comfort, and which exhibits good washability when used as cleansing cosmetics.

### BACKGROUND ART

As a lipopeptide compound derived from a microorganism, for example, sodium surfactin is known as a raw material for cosmetics, which, for its low skin irritating property and favorable biodegradability, has attracted attention, and is disclosed in e.g., WO99/62482 and JP-A-2003-176211.

However, although the cleansing cosmetic disclosed in JP-A-2003-176211 is excellent in cleansing ability of a cosmetic, its washability with water is insufficient.

In particular, no cleansing cosmetic in gel form or cream form having a good washability has been provided.

### DISCLOSURE OF THE INVENTION

An object of the present invention is to provide a cosmetic which, being stable and having an extremely low skin irritating property, ensures high skin comfort, and exhibits a good washability when used in a cleansing cosmetic.

As a result of intensive investigations to solve this problem, the present inventors have found that the aforementioned object is attained by a cosmetic which comprises a lipopeptide compound, and a polyoxyethylene glyceryl ether fatty acid ester and/or a polyoxyethylene sorbit fatty acid ester. Thus, the present invention was accomplished.

Accordingly, the invention relates to the following items.
1. A cosmetic composition, comprising 0.1 to 5 mass% of a lipopeptide compound, and 0.1 to 20 mass% of a polyoxyethylene glyceryl ether fatty acid ester and/or a polyoxyethylene sorbit fatty acid ester.
2. The cosmetic composition according to the above item 1, wherein the lipopeptide compound is derived from a microorganism.
3. The cosmetic composition according to the above item 2, wherein the lipopeptide compound is a surfactin represented by the following formula (I), an analogous compound or a salt thereof: 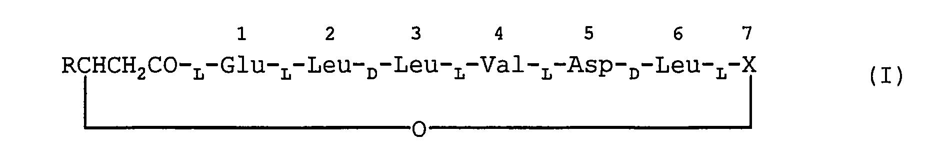 wherein X represents an amino acid residue selected from the group consisting of leucine, isoleucine, valine, glycine, serine, alanine, threonine, asparagine, glutamine, aspartic acid, glutamic acid, lysine, arginine, cysteine, methionine, phenylalanine, tyrosine, tryptophan, histidine, proline, 4-hydroxyproline and homoserine, and R is a normalalkyl group having 8 to 14 carbon atoms, an isoalkyl group having 8 to 14 carbon atoms or an anteiso-alkyl group having 8 to 14 carbon atoms.
4. The cosmetic composition according to the above item 3, wherein the lipopeptide compound is sodium surfactin.
5. The cosmetic composition according to the above item 3, wherein the lipopeptide compound is a compound where the second, fourth and sixth amino acids in the formula (I) are each independently substituted by amino acid selected from a group consisting of leucine, isoleucine, valine, glycine, serine, alanine, threonine, asparagine, glutamine, aspartic acid, glutamic acid, lysine, arginine, cysteine, methionine, phenylalanine, tyrosine, tryptophan, histidine, proline, 4-hydroxyproline and homoserine.
6. A cleansing cosmetic using the cosmetic composition according to any one of the above items 1 to 5.
7. The cleansing cosmetic according to the above item 6, wherein the cosmetic is in gel form or cream form.

### DETAILED DESCRIPTION OF THE INVENTION

The invention is explained below in detail.

Examples of the lipopeptide compound used in the invention include lipopeptide compounds produced by a microorganism of genus *Bacillus* such as *Bacillus subtilis* described in JP-A-2000-327591. Preferable examples include salts of surfactin and salts of an analogous compound thereof. The lipopeptide compound is usually a compound derived from a microorganism.

The surfactin herein refers to a compound represented by the formula (I): 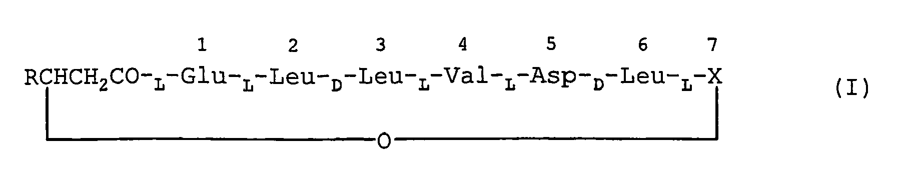 or a composition containing two or more kinds of the compounds represented by the formula (I).

In the above formula (I), X represents an amino acid residue selected from the group consisting of leucine, isoleucine, valine, glycine, serine, alanine, threonine, asparagine, glutamine, aspartic acid, glutamic acid, lysine, arginine, cysteine, methionine, phenylalanine, tyrosine, tryptophan, histidine, proline, 4-hydroxyproline and homoserine. Preferred X is leucine, isoleucine or valine.

R is a normalalkyl group having 8 to 14 carbon atoms, an isoalkyl group having 8 to 14 carbon atoms or an anteiso-alkyl group having 8 to 14 carbon atoms. The normalalkyl group is a straight chain alkyl group; the isoalkyl group usually has a structure which comprises (CH₃)₂CH-(CH₂)ₙ-; and the anteiso-isoalkyl group usually has a structure which comprises CH₃-CH₂-CH(CH₃)-(CH₂)ₙ-.

The analogous compound of surfactin refers to compounds having amino acid(s) substituted by other amino acid (s) in the aforementioned formula (I). Specifically, examples of such a compound include compounds where L-leucine as the second amino acid, L-valine as the fourth amino acid and D-leucine as the sixth amino acid are each independently substituted by amino acid selected from a group consisting of leucine, isoleucine, valine, glycine, serine, alanine, threonine, asparagine, glutamine, aspartic acid, glutamic acid, lysine, arginine, cysteine, methionine, phenylalanine, tyrosine, tryptophan, histidine, proline, 4-hydroxyproline and homoserine, but not limited thereto. Hereinafter, "surfactin or an analogous compound thereof" may be referred to as "surfactin".

Surfactin can be utilized as the inorganic salt or the organic salt as is seen from the above formula (I). Metal used for counter ion may be of any kind, for example, alkali metals such as sodium, potassium and lithium and alkaline earth metals such as calcium and magnesium, as long as the metal forms a salt with surfactin.

Examples of the organic salt include trimethylamine, triethylamine, tributylamine, monoethanolamine, diethanolamine, triethanolamine, lysine, arginine and choline.

Among these, sodium, potassium, monoethanolamine, diethanolamine, triethanolamine, lysine or arginine is preferred, and sodium is particularly preferred.

Sodium surfactin is on the market from SHOWA DENKO K.K., under the trade name of Aminofect (registered trademark of SHOWA DENKO K.K.).

The amount of lipopeptide compound contained in the cosmetic of the invention is preferably 0.1 to 5 mass%, more preferably 0.2 to 2 mass%, and still more preferably 0.4 to 1 mass%. When the amount is less than 0.1 mass%, the stability of the cosmetic may be insufficient, and also in cases where the compound is used in an amount exceeding 5 mass%, the stability may rather deteriorate.

The polyoxyethylene glyceryl ether fatty acid ester and/or polyoxyethylene sorbit fatty acid ester used in the invention may may be used without any particular limitation, as far as the compound is conventionally employed in preparation of cosmetics. Examples of the polyoxyethylene glyceryl ether fatty acid ester include polyoxyethylene (3) glyceryl triisostearate, polyoxyethylene (5) glyceryl triisostearate, polyoxyethylene (10) glyceryl triisostearate, polyoxyethylene (20) glyceryl triisostearate, polyoxyethylene (30) glyceryl triisostearate, polyoxyethylene (40) glyceryl triisostearate, polyoxyethylene (50) glyceryl triisostearate, polyoxyethylene (60) glyceryl triisostearate, polyoxyethylene (3) glyceryl isostearate, polyoxyethylene (5) glyceryl isostearate, polyoxyethylene (6) glyceryl isostearate, polyoxyethylene (8) glyceryl isostearate, polyoxyethylene (10) glyceryl isostearate, polyoxyethylene (15) glyceryl isostearate, polyoxyethylene (20) glyceryl isostearate, polyoxyethylene (25) glyceryl isostearate, polyoxyethylene (30) glyceryl isostearate, polyoxyethylene (40) glyceryl isostearate, polyoxyethylene (50) glyceryl isostearate, polyoxyethylene (60) glyceryl isostearate, polyoxyethylene (3) glyceryl tristearate, polyoxyethylene (4) glyceryl tristearate, polyoxyethylene (5) glyceryl tristearate, polyoxyethylene (6) glyceryl tristearate, polyoxyethylene (10) glyceryl tristearate, polyoxyethylene (20) glyceryl tristearate, polyoxyethylene (4) glyceryl distearate, polyoxyethylene (3) glyceryl trioleate, polyoxyethylene (5) glyceryl trioleate, polyoxyethylene (10) glyceryl trioleate, polyoxyethylene (20) glyceryl trioleate, polyoxyethylene (30) glyceryl trioleate, polyoxyethylene (40) glyceryl trioleate, polyoxyethylene (50) glyceryl trioleate and polyoxyethylene (60) glyceryl trioleate. For example, in a case where the compound is used for the purpose of improving washability as a cleansing cosmetic, those having a hydrophile-lipophile balance (HLB) of 6 to 13 are preferred, and among these, polyoxyethylene (20) glyceryl triisostearate is particularly preferred. Examples of the polyoxyethylene sorbit fatty acid ester include polyoxyethylene sorbitmonolaurate, polyoxyethylene (40) sorbit oleate, polyoxyethylene (4) sorbit tetraoleate, polyoxyethylene (3) sorbit tristearate, polyoxyethylene (30) sorbit tetraoleate, polyoxyethylene (40) sorbit tetraoleate, polyoxyethylene (60) sorbit tetraoleate, polyoxyethylene (3) sorbit isostearate, polyoxyethylene (40) sorbit oleate, polyoxyethylene (60) sorbit tetrastearate, polyoxyethylene (6) sorbit hexaoleate, polyoxyethylene sorbit hexastearate and polyoxyethylene (40) sorbit pentaoleate. For example, in a case where the compound is used for the purpose of improving washability as a cleansing cosmetic, for example, those having an HLB of 6 to 13 are preferred, and among these, polyoxyethylene (30) sorbit tetraoleate is particularlypreferred. These maybe used alone or in combination of two or more of them.

The amount of polyoxyethylene glyceryl ether fatty acid ester and/or polyoxyethylene sorbit fatty acid ester contained in the cosmetic of the invention is preferably 0.1 to 20 mass%, more preferably 0.4 to 18 mass%, and still more preferably 1 to 16 mass%. If the amount is less than 0.1 mass%, the object of the invention cannot be achieved in light of the stability, washability and the like properties of the cosmetic, and if the amount exceeds 20 mass%, it is unfavorable in that the cosmetic deteriorates in stability or skin comfort.

The cosmetic of the invention can be produced according to a conventional method. More specifically, it can be prepared using a generally utilized stirring apparatus or a generally utilized emulsification apparatus. Moreover, in instances where a cleansing cosmetic in gel form is produced, it canbe also prepared by such a method as one disclosed in JP-A-2003-176211. Further, examples of the form for use of the cosmetic of the invention include skin toner, lotion, milky lotion, essence, cream, pack, makeup base, makeup cosmetics, massaging cosmetics and cleansing cosmetics. Among them, when a cleansing cosmetic is prepared according to the method as disclosed in JP-A-2003-176211, it is suitable because the property upon washing away with water becomes favorable.

The cosmetic of the invention may contain optional ingredients which are generally used in cosmetics in the range where the object of the invention may be achieved.

Examples of such an ingredient include hydrocarbons such as ozokerite, α-olefin oligomer, light isoparaffin, light liquid isoparaffin, squalene, squalane, synthetic squalane, phytosqualane, ceresin, paraffin, polyethylene powder, polybutene, microcrystalline wax, liquid isoparaffin, liquid paraffin, mineral oil and vaseline;
natural waxes such as jojoba oil, carnauba wax, candelilla wax, rice bran wax, shellac, lanolin, mink sebaceous wax, spermaceti wax, sugarcane wax, sperm whale oil, beeswax andmontan wax, natural fats and fatty oils such as avocado oil, almond oil, olive oil, extra virgin olive oil, sesame seed oil, rice bran oil, rice oil, rice germ oil, corn oil, safflower oil, soybean oil, maize oil, rape seed oil, persic oil, palm kernel oil, palm oil, castor oil, sunflower oil, high oleic sunflower oil, grape seed oil, cotton seed oil, coconut oil, hydrogenated coconut oil, beef tallow, hydrogenated oil, horse oil, mink oil, yolk oil, yolk fat oil, rose hip oil, kukui nut oil, evening primrose oil, wheat germ oil, peanut oil, Camellia jeponica oil, Camellia kissi oil, cacao butter, Japan wax, beef bone tallow, nest's-foot oil, swine tallow, equine tallow, ovine tallow, shea butter, macadamia nut oil and meadowfoam seed oil;
fatty acids such as lauric acid, myristic acid, palmitic acid, stearic acid, behenic acid, oleic acid, linoleic acid, linolenic acid, γ-linolenic acid, isostearic acid, 12-hydroxystearic acid, undecylenic acid and coconut oil fatty acid;
higher alcohols such as isostearyl alcohol, octyl dodecanol, hexyl decanol, cholesterol, phytosterol, lauryl alcohol, myristyl alcohol, cetyl alcohol, stearyl alcohol, oleyl alcohol, behenyl alcohol and cetostearyl alcohol;
alkylglyceryl ethers such as batyl alcohol, chimylalcohol, serachyl alcohol and isostearyl glyceryl ether;
esters such as isopropyl myristate, butyl myristate, isopropylpalmitate, ethyl stearate, butyl stearate, ethyl oleate, ethyl linoleate, isopropyl linoleate, cetyl caprylate, hexyl laurate, isooctylmyristate, decylmyristate, myristylmyristate, cetyl myristate, octadecyl myristate, cetyl palmitate, stearyl stearate, decyl oleate, oleyl oleate, cetyl ricinoleate, isostearyl laurate, isotridecyl myristate, isocetyl myristate, isostearyl myristate, octyldodecyl myristate, 2-ethylhexyl palmitate, isocetyl palmitate, isostearyl palmitate, 2-ethylhexyl stearate, isocetyl stearate, isodecyl oleate, octyldodecyl oleate, octyldodecyl ricinoleate, ethyl isostearate, isopropyl isostearate, cetyl 2-ethylhexanoate, cetostearyl 2-ethylhexanoate, stearyl 2-ethylhexanoate, hexyl isostearate, ethylene glycol dioctanoate, ethylene glycol dioleate, propylene glycol dicaprylate, propylene glycol dicaprylate/dicaprate, propylene glycol dicaprate, propylene glycol dioleate, neopentyl glycol dicaprate, neopentyl glycol dioctanoate, glyceryl tricaprylate, glyceryl tri 2-ethyl hexanoate, glyceryl tricaprylate/tricaprate, glyceryl tricaprylate/tricaprate/tristearate, glyceryl triundecylate, glyceryl triisopalmitate, glyceryl triisostearate, trimethylolpropane tri 2-ethylhexanoate, trimethylolpropane triisostearate, pentaerythrityl tetra 2-ethylhexanoate, pentaerythrityl tetramyristate, pentaerythrityl tetraisostearate, diglyceryl tetraisostearate, octyldodecyl neopentanotae, isocetyl octanoate, isostearyl octanoate, 2-ethylhexyl isopelargonate, hexyldecyl dimethyloctanoate, octyldodecyl dimethyloctanoate, 2-ethylhexyl isopalmitate, isocetyl isostearate, isostearyl isostearate, octyldodecyl isostearate, lauryl lactate, myristyl lactate, cetyl lactate, octyldodecyllactate, triethyl citrate, acetyltriethyl citrate, acetyltributyl citrate, trioctyl citrate, triisocetyl citrate, trioctyldodecyl citrate, diisostearyl malate, 2-ethylhexyl hydroxystearate, di 2-ethylhexyl succinate, diisopropyl adipate, diisobutyl adipate, dioctyl adipate, diheptylundecyl adipate, sebacate diethyl, diisopropyl sebacate, dioctyl sebacate, cholesteryl stearate, cholesteryl isostearate, cholesteryl hydroxystearate,cholesteryloleate,dihydrocholesteryl oleate, phytosteryl isostearate, phytosteryl oleate, isocetyl 12-stearoyl hydroxystearate, stearyl 12-stearoyl hydroxystearate, isostearyl 12-stearoyl hydroxystearate, polyoxyethylene (3) polyoxypropylene (1) cetyl ether acetate, polyoxyethylene (3) polyoxypropylene (1) isocetyl ether acetate, isononyl isononanoate, octyl isononanoate, tridecyl isononanoate and isotridecyl isononanoate;
silicone oils such as methyl polysiloxane, methylphenyl polysiloxane, methyl hydrogen polysiloxane, methyl cyclopolysiloxane, octamethyl cyclotetrasiloxane, decamethyl cyclopentasiloxane, dodecamethyl cyclohexasiloxane, octamethyl trisiloxane, decamethyl tetrasiloxane, tetradecamethyl hexasiloxane, highly polymerized methyl polysiloxane, dimethylsiloxane-methyl (polyoxyethylene) siloxane-methyl (poly oxypropylene)siloxane copolymer, dimethylsiloxane-methyl(polyoxyethylene)siloxane copolymer, dimethylsiloxane-methyl(polyoxypropylene)siloxane copolymer, dimethylsiloxane-methylcetyl oxysiloxane copolymer, dimethylsiloxane-methyl stearoxysiloxane copolymer, polyether modified silicone, alcohol modified silicone, alkyl modified silicone and amino modified silicone;
polyhydric alcohols such as ethylene glycol, diethylene glycol, triethylene glycol, polyethylene glycol, propylene glycol, dipropylene glycol, polypropylene glycol, glycerin, diglycerin, polyglycerin, 3-methyl-1,3-butanediol, 1,3-butanediol, 1,2-pentanediol and 1,2-hexanediol;
saccharidessuch asmannitol, sorbitol, xylitol, maltitol, erythritol, pentaerythritol, glucose, sucrose, fructose, lactose, maltose, xylose and trehalose;
polymers such as sodium alginate, carrageen, agar, furcellaran, guar gum, quince seed, Amorphophalus konjak (arum root) mannan, tamarind gum, tara gum, dextrin, starch, locust bean gum, gum arabic, gum gatti, karaya gum, gum tragacanth, arabinogalactan, pectin, quince, chitosan, starch, curdlan, xanthan gum, gellan gum, cyclodextrin, dextran, pullulan, microcrystalline cellulose, methyl cellulose, ethyl cellulose, hydroxyethyl cellulose, hydroxypropyl cellulose, hydroxypropylmethyl cellulose, carboxymethyl cellulose, carboxy starch, cationized cellulose, starch phosphate ester, cationized guar gum, carboxymethyl-hydroxypropylated guar gum, hydroxypropylated guar gum, albumin, casein, gelatin, sodium polyacrylate, polyacrylic amide, carboxyvinyl polymer, polyethylene imine, highly polymerized polyethylene glycol, polyvinyl alcohol, polyvinyl pyrrolidone, polyvinyl ether, polyacryl amide, acrylic acid copolymer, methacrylic acid copolymer, maleic acid copolymer, vinylpyridine copolymer, ethylene/acrylic acid copolymer, vinyl pyrrolidone based polymer, vinylalcohol/vinyl pyrrolidone copolymer, nitrogen-substituted acrylamide based polymer, amino modified silicone, cationized polymer, dimethylacryl ammoniumbasedpolymer, acrylic acidbased anion polymer, methacrylic acid based anion polymer, modified silicone, acrylate/methacrylate alkyl (C 10 to 30) copolymer and polyoxyethylene/polyoxypropylene copolymer;
alcohols such as ethanol, isopropyl alcohol, 1-butanol, 2-butanol and benzyl alcohol;
anionic surfactants such as coconut oil fatty acidpotassium, coconut oil fatty acid sodium, coconut oil fatty acid triethanolamine, potassium laurate, sodium laurate, triethanolamine laurate, potassiummyristate, sodiummyristate, isopropanolamine myristate, potassium palmitate, sodium palmitate, isopropanolamine palmitate, potassium stearate, sodium stearate, triethanolamine stearate, potassium oleate, sodium oleate, castor oil fatty acid sodium, zinc undecylate, zinc laurate,zinc myristate,magnesium myristate, zincpalmitate, zinc stearate, calcium stearate, magnesium stearate, aluminum stearate, calcium myristate, magnesium myristate, aluminum dimyristate, aluminum isostearate, polyoxyethylene lauryl ether acetate, sodium polyoxyethylene lauryl ether acetate, polyoxyethylene tridecyl ether acetate, sodium polyoxyethylene tridecyl ether acetate, sodium stearoyl lactate, sodium isostearoyl lactate, sodium lauroyl sarcosine, coconut oil fatty acid sarcosine, sodium coconut oil fatty acid sarcosine, coconut oil fatty acid sarcosine triethanolamine, lauroyl sarcosine, potassium lauroyl sarcosine, lauroyl sarcosine triethanolamine, oleoyl sarcosine, sodium myristoyl sarcosine, sodium stearoyl glutamate, coconut oil fatty acid acyl glutamic acid, potassium coconut oil fatty acid acyl glutamate, sodium coconut oil fatty acid acyl glutamate, coconut oil fatty acid acyl glutamate triethanolamine, lauroylacyl glutamic acid, potassium lauroylacyl glutamate, sodium lauroylacyl glutamate, lauroylacyl glutamate triethanolamine, myristoylacyl glutamic acid, potassium myristoylacyl glutamate, sodium myristoylacyl glutamate, stearoylacyl glutamic acid, potassium stearoylacyl glutamate, disodiumstearoylacyl glutamate, sodium hydrogenated beef tallow fatty acid acyl glutamate, sodium coconut oil fatty acid/hydrogenated beef tallow fatty acid acyl glutamate, sodium coconut oil fatty acid methylalanine, lauroyl methylalanine, sodium lauroyl methylalanine, lauroyl methylalanine triethanolamine, sodiummyristoyl methylalanine, sodium lauroyl methyltaurine, potassium coconut oil fatty acid methyltaurine, sodium coconut oil fatty acid methyltaurine, magnesium coconut oil fatty acid methyltaurine, sodium myristoyl methyltaurine, sodium palmitoyl methyltaurine, sodium stearoyl methyltaurine, sodium oleoyl methyltaurine, sodium alkane sulfonate, sodium tetradecene sulfonate, sodium sulfosuccinate dioctyl, disodium lauryl sulfosuccinate, sodium coconut oil fatty acid ethyl ester sulfonate, sodium lauryl sulfate, triethanolamine lauryl sulfate, sodium cetyl sulfate, triethanolamine alkyl (11, 13, 15) sulfate, sodium alkyl (12,13) sulfate, triethanolamine alkyl (12,13) sulfate, alkyl (12, 14, 16) ammonium sulfate, diethanolamine alkyl (12 to 13) sulfate, triethanolamine alkyl (12 to 14) sulfate, triethanolamine alkyl (12 to 15) sulfate, magnesium coconut oil alkyl sulfate/triethanolamine, lauryl ammonium sulfate, potassium lauryl sulfate, magnesium lauryl sulfate, monoethanolamine lauryl sulfate, diethanolamine lauryl sulfate, sodium myristyl sulfate, sodium stearylsulfate, sodium oleyl sulfate, triethanolamine oleyl sulfate, sodium polyoxyethylene lauryl ether sulfate, triethanolamine polyoxyethylene lauryl ether sulfate, sodium polyoxyethylene (1) alkyl (11,13,15) ether sulfate, triethanolamine polyoxyethylene (1) alkyl (11,13,15) ether sulfate, sodium polyoxyethylene (3) alkyl (11 to 15) ether sulfate, sodiumpolyoxyethylene (2) alkyl (12,13) ether sulfate, sodium polyoxyethylene (3) alkyl (12 to 14) ether sulfate, sodium polyoxyethylene (3) alkyl (12 to 15) ether sulfate, sodium polyoxyethylene (2) lauryl ether sulfate, sodiumpolyoxyethylene (3) myristyl ether sulfate, sodium higher fatty acid alkanol amide sulfate ester, lauryl phosphate, sodium lauryl phosphate, potassium cetyl phosphate, diethanolamine cetyl phosphate, polyoxyethylene oleyl ether phosphate, polyoxyethylene lauryl ether phosphate, sodium polyoxyethylene lauryl ether phosphate, polyoxyethylene cetyl ether phosphate, sodium polyoxyethylene cetyl ether phosphate, polyoxyethylene stearyl ether phosphate, polyoxyethylene oleyl ether phosphate, sodium polyoxyethylene oleyl ether phosphate, polyoxyethylene alkylphenyl ether phosphate, sodium polyoxyethylene alkylphenyl ether phosphate, triethanolamine polyoxyethylene alkylphenyl ether phosphate, polyoxyethylene octyl ether phosphate, polyoxyethylene (10) alkyl (12,13) ether phosphate, polyoxyethylene alkyl (12 to 15) etherphosphate, polyoxyethylene alkyl (12 to 16) etherphosphate, triethanolamine polyoxyethylene lauryl ether phosphate and diethanolamine polyoxyethylene oleyl ether phosphate;
cationic surfactants such as dioctylamine, dimethylstearylamine, trilaurylamine, diethylaminoethylamide stearate, lauryl trimethylammonium chloride, cetyl trimethylammonium chloride, cetyl trimethylammonium bromide, cetyl trimethylammonium saccharin, stearyl trimethylammonium chloride, alkyl (20 to 22) trimethylammonium chloride, lauryl trimethylammonium bromide, alkyl (16,18) trimethylammonium chloride, stearyl trimethylammonium bromide, stearyl trimethylammonium saccharin, alkyl (28) trimethylammonium chloride, di(polyoxyethylene) oleyl methylammonium (2EO) chloride, dipolyoxyethylene stearyl methylammonium chloride, polyoxyethylene (1) polyoxypropylene (25) diethylmethylammonium chloride, tri (polyoxyethylene) stearyl ammonium (5EO) chloride, distearyl dimethylammonium chloride, dialkyl (12 to 15) dimethylammonium chloride, dialkyl (12 to 18) dimethylammonium chloride, dialkyl (14 to 18) dimethylammonium chloride, dicocoyl dimethylammonium chloride, dicetyl dimethylammonium chloride, isostearyllauryl dimethylammonium chloride, benzalkonium chloride, myristyl dimethylbenzyl ammonium chloride, lauryl dimethyl (ethylbenzyl) ammonium chloride, stearyl dimethylbenzyl ammonium chloride, lauryl pyridinium chloride, cetyl pyridinium chloride, lauroyl cholamino formylmethyl pyridinium chloride, stearoyl cholamino formylmethyl pyridinium chloride, alkyl isoquinolinium bromide, methyl benzethonium chloride and benzethonium chloride;
ampholytic surfactants such as 2-alkyl-N-carboxymethyl-N-hydroxyethyl imidazolium betaine, alkyldiamino ethyl glycine hydrochloride, sodium lauryldiamino ethyl glycine, sodium undecyl hydroxyethyl imidazolium betaine, undecyl-N-carboxymethyl imidazolium betaine, disodium coconut oil fatty acid acyl-N-carboxyethyl-N-hydroxyethyl ethylenediamine, disodium coconut oil fatty acid acyl-N-carboxyethoxyethyl-N-carboxyethyl ethylenediamine, disodium coconut oil fatty acid acyl-N-carboxymethoxyethyl-N-carboxymethyl ethylenediamine, sodium laurylamino propionate, sodium laurylamino dipropionate, triethanolamine laurylamino propionate, sodium palm oil fatty acid acyl-N-carboxyethyl-N-hydroxyethyl ethylenediamine, betaine lauryldimethylamino acetate, betaine coconut oil alkyldimethylamino acetate, betaine stearyl dimethylamino acetate, sodium stearyldimethyl betaine, coconut oil fatty acid amidopropyl betaine, palm oil fatty acid amidopropyl betaine, amidopropyl acetate betaine laurate, amidopropyl betaine ricinoleate, stearyl dihydroxyethyl betaine and lauryl hydroxysulfobetaine;
nonionic surfactants such as polyoxyethylene (10) alkyl (12,13) ether, polyoxyethylene lauryl ether, polyoxyethylene cetyl ether, polyoxyethylene stearyl ether, polyoxyethylene oleyl ether, polyoxyethylene (3,7,12) alkyl (12 to 14) ether, polyoxyethylene tridecyl ether, polyoxyethylene myristyl ether, polyoxyethylene-sec-alkyl (14) ether, polyoxyethylene isocetyl ether, polyoxyethylene cetostearyl ether, polyoxyethylene (2,10,20) isostearyl ether, polyoxyethylene oleylcetyl ether, polyoxyethylene (20) arachyl ether, polyoxyethylene octyldodecyl ether, polyoxyethylene behenyl ether, polyoxyethylene octylphenyl ether, polyoxyethylene nonylphenyl ether, polyoxyethylene dinonylphenyl ether, polyoxyethylene (1) polyoxypropylene (1,2,4,8) cetyl ether, polyoxyethylene (5) polyoxypropylene (1,2,4,8) cetyl ether, polyoxyethylene (10) polyoxypropylene (1,2,4,8) cetyl ether, polyoxyethylene (20) polyoxypropylene (1,2,4,8) cetyl ether, polyoxyethylene polyoxypropylene lauryl ether, polyoxyethylene (3) polyoxypropylene (34) stearyl ether, polyoxyethylene (4) polyoxypropylene (30) stearyl ether, polyoxyethylene (34) polyoxypropylene (23) stearyl ether, polyoxyethylene polyoxypropylene cetyl ether, polyoxyethylene polyoxypropylene decyltetradecyl ether, polyethylene glycol monolaurate, ethylene glycol monostearate, polyethylene glycol monostearate, polyethylene glycolmonooleate, ethylene glycol fatty acid ester, self-emulsifying ethylene glycol monostearate, diethylene glycol laurate, polyethylene glycol myristate, polyethylene glycol palmitate, diethylene glycol stearate, self-emulsifying polyethylene glycol (2) monostearate, polyethylene glycol isostearate, ethylene glycol dioctanoate, diethylene glycol dilaurate, polyethylene glycol dilaurate, polyethylene glycol (150) dipalmitate, ethylene glycol distearate, diethylene glycol distearate, polyethylene glycol distearate, ethylene glycol dioleate, polyethylene glycol dioleate, polyethylene glycol diricinoleate, polyoxyethylene (20) sorbitan monolaurate, polyoxyethylene (20) sorbitan monopalmitate, polyoxyethylene (6) sorbitan monostearate, polyoxyethylene (20) sorbitan monostearate, polyoxyethylene (20) sorbitan tristearate, polyoxyethylene (6) sorbitan monooleate, polyoxyethylene (20) sorbitan monooleate, polyoxyethylene (20) sorbitan trioleate, polyoxyethylene (20) coconut oil fatty acid sorbitan, polyoxyethylene (10 to 80) sorbitanmonolaurate, polyoxyethylene sorbitan tristearate, polyoxyethylene (20) sorbitan isostearate, polyoxyethylene (150) sorbitan tristearate, polyoxyethylene castor oil, polyoxyethylene hydrogenated castor oil, polyoxyethylene (10) hydrogenated castor oil, polyoxyethylene (20) hydrogenated castor oil, polyoxyethylene (40) hydrogenated castor oil, polyoxyethylene (50) hydrogenated castor oil, polyoxyethylene (60) hydrogenated castor oil, lipophilic glycerin monostearate, lipophilic glycerin monooleate, self-emulsifying glycerin monostearate, coconut oil fatty acid glyceryl, glycerin laurate, glyceryl myristate, glyceryl isostearate, glyceryl ricinoleate, glyceryl monohydroxystearate, glycerin oleate, glyceryl linoleate, glyceryl erucate, glyceryl behenate, wheat germoil fatty acid glyceride, safflower oil fatty acid glyceryl, hydrogenated soybean fatty acid glyceryl, saturated fatty acid glyceride, cotton seed oil fatty acid glyceryl, monomyristate glyceryl monoisostearate, mono tallowate glyceride, monolanolin fatty acid glyceryl, glyceryl sesquioleate, glyceryl distearate, glyceryl diisostearate, glyceryl diarachidate, sorbitan monolaurate, sorbitan monopalmitate, sorbitan monostearate, sorbitan monoisostearate, sorbitan monooleate, sorbitan sesquistearate, sorbitan sesquioleate, sorbitan tristearate, sorbitan trioleate, coconut oil fatty acid sorbitan, sorbitan isostearate, sorbitan sesquiisostearate, sorbitan distearate, diglyceryl isopalmitate, poly(4 to 10)glyceryl monolaurate, poly(10)glyceryl monomyristate, poly(2 to 10)glyceryl monostearate, poly(2 to 10) glyceryl monoisostearate, poly (2 to 10) glyceryl monooleate, diglyceryl sesquioleate, poly(2 to 10)glyceryl diisostearate, poly(6 to 10)glyceryl distearate, diglyceryl triisostearate, poly(10)glyceryl tristearate, poly(10)glyceryl trioleate, poly(2)glyceryl tetraisostearate, decaglyceryl pentastearate, poly(6 to 10)glyceryl pentaoleate, poly(10)glyceryl heptastearate, decaglyceryl decastearate, poly(10)glyceryl decaoleate, concentrated poly(6)glyceryl ricinoleate, sucrose fatty acid ester, coconut oil fatty acid sucrose ester, alkyl glucoside, coconut oil alkyl dimethylamine oxide, lauryl dimethylamine oxide, dihydroxyethyl lauryl dimethylamine oxide, stearyl dimethylamine oxide, oleyl dimethylamine oxide and polyoxyethylene coconut oil alkyl dimethylamine oxide;
natural surfactants such as saponin, lecithin, soybean phospholipid, hydrogenated soybean phospholipid, soybean lysophospholipid, hydrogenated soybean lysophospholipid, yolk lecithin, hydrogenated yolk lysophosphatidylcholine, phosphatidylcholine, phosphatidylethanolamine, phosphatidylserine, sphingophospholipid, sphingomyelin, ganglioside, bile acid, cholic acid, deoxycholic acid, sodium cholate, sodium deoxycholate, spiculisporic acid, rhamnolipid, trehalose lipid, sophorolipid and mannosyl erythritol lipid;
ultraviolet ray absorbers such as: para-aminobenzoic acid derivatives such as para-aminobenzoic acid, ethyl para-aminobenzoate, glyceryl para-aminobenzoate, amyl para-dimethyl aminobenzoate and 2-ethylhexyl para-dimethyl aminobenzoate; cinnamic acid derivatives such as benzyl cinnamate, mono-2-ethyl hexanoate glyceryl dipara-methoxycinnamate, methyl 2,4-diisopropyl cinnamate, ethyl 2,4-diisopropyl cinnamate, potassium para-methoxycinnamate, sodium para-methoxycinnamate, isopropyl para-methoxycinnamate, 2-ethylhexyl para-methoxycinnamate, 2-ethoxyethyl para-methoxycinnamate and ethyl para-ethoxycinnamate; urocanic acid derivatives such as urocanic acid and ethyl urocanate; benzophenone derivatives such as 2,4-dihydroxybenzophenone, 2,2',4,4'-tetrahydroxybenzophenone, sodium 2-hydroxy-4-methoxy-5-sulfobenzophenone, 2-hydroxy-4-methoxybenzophenone-5-sulfonate, 2-hydroxy-4-methoxybenzophenone, 2,2'-dihydroxy-4,4'-dimethoxybenzophenone and sodium 2,2'-dihydroxy-4,4'-dimethoxy-5-sulfobenzophenone; salicylic acid derivatives such as ethylene glycol salicylate, salicylate-2-ethylhexyl, phenyl salicylate, benzyl salicylate, p-tert-butylphenyl salicylate, homomenthyl salicylate and salicylate-3,3,5-trimethylcyclohexyl; 2-(2'-hydroxy-5'-methoxyphenyl)benzotriazole and 4-tert-butyl-4'-methoxybenzoyl methane;
powders and color materials such as: kaolin, silicic anhydride, magnesium aluminum silicate, sericite, talc, boron nitride, mica, montmorillonite, hemp cellulose powder, wheat starch, silk powder, maize starch; natural dyes such as nitro dyes, azo dyes, nitroso dyes, triphenylmethane dyes, xanthene dyes, quinoline dyes, anthraquinone dyes, indigo dyes, pyrene dyes, phthalocyanine dyes, flavonoid, quinone, porphyrin, water soluble annatto, sepia powder, caramel, guaiazulene, gardenia blue, gardenia yellow, cochineal, shikonin, sodium copper chlorophyllin, paprika dye, safflower red, safflower yellow, laccaic acid and riboflavin butyrate ester; carbon black, yellow iron oxide, black iron oxide, red iron oxide, iron blue, ultramarine blue, zinc oxide, chromium oxide, titanium oxide, black titanium oxide, zirconium oxide, chromium hydroxide, alumina, magnesium oxide, barium sulfate, aluminum hydroxide, calcium carbonate, lithium cobalt titanate, manganese violet and pearl pigment.
plant extracts such as Angelica keiskei extract, Uncaria gambir extract, avocado extract, sweet hydrangea leaf extract, Gynostemma pentaphyllum makino extract, Althaea officinalis extract, Arnica montana extract, oil soluble Arnica montana extract, almond extract, aloe extract, Japanese styrax benzoin extract, Ginkgo biloba extract, Stinging nettle extract, Orris rhizome root extract, fennel extract, turmeric extract, dog rose fruit extract, Echinacea leaf extract, Scutellaria root extract, Phellodendron bark extract, Japanese captis extract, barley extract, okura extract, Hypericum perforatum extract, oil soluble Hypericum perforatum extract, Lamium album extract, oil soluble Lamium album extract, Ononis spinosa root extract, Nasturtium officinale extract, orange extract, orange flower water, seaweed extract, persimmon tannin, pueraria root extract, Japanese valerian extract, cattail extract, Chamomile(matricaria) extract, oil soluble Chamomile (matricaria) extract, Chamomile (matricaria) distillate, Avena sativa (oat) kernel extract, carrot extract, oil soluble carrot extract, carrot oil, Artemisia capillaris extract, Glycyrrhiza glabra (licorice) extract, powdered Glycyrrhiza glabra (licorice) extract, Glycyrrhiza glabra (licorice) extract flavonoid, cantharides tincture, raspberry extract, kiwi extract, cinchona extract, cucumber extract, apricot kernel extract, quince seed extract, gardenia florida extract, Sasaalbomarginata extract, Sophoraroot extract, walnut shell extract, Citrus paradisi (grapefruit) extract, Clematis vitalba leaf extract, black sugar extract, chlorella extract, mulberry bark extract, Cinnamon bark extract, Gentian extract,Geranium herb extract,black tea extract, Nuphar extract, burdock root extract, oil soluble burdock root extract, wheat germ extract, hydrolyzed wheat powder, rice bran extract, fermented rice bran extract, Symphytum officinale (comfrey) extract, Asiasarum root extract, Crocus sativus (saffron) extract, Saponaria officinalis extract, oil soluble salvia extract, Crataegus cuneata fruit extract, Zanthoxylum fruit extract, Lentinus edodes extract, powdered Lentinus edodes extract, Rehmannia root extract, Lithospermum root extract, oil soluble Lithospermum root extract, Perilla herb extract, linden extract, oil soluble Tilia europaea extract, Filipendula extract, Peony root extract, Coix lacryma-jobi extract, ginger extract, oil soluble ginger extract, ginger tincture, Acorus calamus root extract, Betula pendula (birch) extract, oil soluble Betula alba (birch) extract, Betula pendula (birch) sap, Lonicera japonica extract, Equisetum arvense extract, oil soluble Equisetum arvense extract, scordinin, stevia extract, ivy extract, Crataegus oxyacantaha (whitethorn) extract, sambucus extract, Juniperus communis extract, Achillea milefolium extract, oil soluble Achillea milefolium extract, Mentha piperita (peppermint) extract, Salvia officinalis (sage) extract, oil soluble Salvia officinalis (sage) extract, Salvia officinalis (sage) water, Malva Sylvestris (mallow) extract, Apium graveolens (celery) extract, Cnidium officinale extract, Cnidium officinale water, Swertia herb extract, Glycine max (soybean) extract, Jujube extract, thyme extract, green tea extract, tea leaf dry distilled solution, tea seed extract, clove extract, Citrus unshiu peel extract, Camellia japonica extract, Centella asiatica extract, oil soluble walnut extract, duku extract, Terminalia sericea extract, Capsicum tincture, Japanese angelica root extract, oil soluble Japanese angelica root extract, Japanese angelica root water, Calendula officinalis flower extract, oil soluble Calendula officinalis flower extract, soy milk powder, peach seed extract, Bitter orange peel extract, Houttuynia cordata extract, Solanum lycopersicum (tomato) extract, Potentilla tormentilla Schrk (Rosaceae) extract, fermented soybeans extract, Ginseng extract, oil soluble Ginseng extract, Allium sativum (garlic) extract, wild rose extract, oil soluble wild rose extract, malt extract, malt root extract, Ophiopogon tuber extract, parsley extract, rye leaf juice concentrate, peppermint distillate, witch hazel distillate, witch hazel extract, rose extract, parietaria extract, Isodonis japonicus extract, Eriobotrya japonica leaf extract, oil soluble Eriobotrya japonica leaf extract, coltsfoot extract, hoelen extract, Ruscus aculeatus root extract, powdered Ruscus aculeatus root extract, grape extract, grape leaf extract, grape water, Hayflower extract, Luffa cylindrica fruit extract, Luffa cylindrica fruit water, Carthamus tinctorius (safflower) extract, oil soluble Tilia platyphyllos extract, linden distillate, Paeonia suffruticosa (peony) extract, Humulus lupulus (hops) extract, oilsolubleHumuluslupulus (hops) extract, pine extract, Silybum marianum (milk thistle) extract, Aesculus hippocastanum (horse chestnut) extract, oil soluble Aesculus hippocastanum (horse chestnut) extract, Sapindus mukurossi extract, Melissa officinalis (balm mint) extract, Melilotus officinalis (melilot) extract, Prunus persica(peach)leaf extract, oil soluble Prunus persica(peach)leaf extract, bean sprouts extract, Centaurea cyanus flower extract, Centaurea cyanus flower distillate, Eucalyptus globulus extract, Saxifrage extract, Lilium (lily) extract, Coix seed extract, oil soluble Coix seed extract, Artemisia princeps pampanini extract, Artemisia princeps pampanini water, Lavandula angustifolia (lavender) extract, Lavandula angustifolia (lavender) water, apple extract, Ganoderma lucidum extract, Lactuca sativa (lettuce) extract, lemon extract, Astragalus sinicus extract, Rosa centifolia (rose) flower water, Rosemarinus officinalis (rosemary) extract, oil soluble Rosemarinus officinalis (rosemary) extract, Anthemis nobilis extract and Sanguisorba officinalis extract;
amino acids and peptides such as glycine, alanine, valine, leucine, isoleucine, serine, threonine, phenylalanine, tyrosine, tryptophan, cystine, cysteine, methionine, proline, hydroxyproline, aspartic acid, asparagine, glutamic acid, glutamine, arginine, histidine, lysine, γ-aminobutyric acid, DL-pyrrolidonecarboxylic acid, ε-aminocaproic acid, hydrolyzed elastin, water soluble elastin, hydrolyzed collagen, water soluble collagen, casein, glutathione, wheat peptides and soybean peptide;
vitamins and factors acting like a vitamin such as: vitamin A and analogues thereof such as retinol, retinal, retinoic acid, retinol acetate and retinol palmitate; carotenoids such as α-carotene, β-carotene, γ-carotene, δ-carotene, lycopene, zeaxanthin, cryptoxanthin, echinenon and astaxanthin; vitamin B₁ and analogues thereof such as thiamines; vitamin B₂ and analogues thereof such as riboflavin; vitamin B₆ and analogues thereof such as pyridoxine, pyridoxal and pyridoxamine; vitamin B₁₂ and analogues thereof such as cyanocobalamin; folic acids, nicotinic acid, nicotinamide, pantothenic acids, biotins; vitamin C and analogues thereof such as L-ascorbic acid, sodium L-ascorbate, L-ascorbyl stearate, L-ascorbyl palmitate, L-ascorbyl dipalmitate, L-ascorbyl tetraisopalmitate, L-ascorbate sulfate disodium ester, magnesium L-ascorbyl, sodium L-ascorbyl phosphate and L-ascorbate-2-glucoside; vitamin D and analogues thereof such as ergocalciferol and cholecalciferol; vitamin E and analogues thereof such as d-α-tocopherol, DL-α-tocopherol, dl-α-tocopherol acetate, dl-α-tocopherol succinate, β-tocopherol, γ-tocopherol and d-δ-tocopherol; ubiquinones, vitamin K and analogues thereof, carnitine, ferulic acid, γ-oryzanol, α-lipoic acid and orotic acid;
antiseptic agents such as benzoic acid, sodium benzoate, undecylenic acid, salicylic acid, sorbic acid, potassiumsorbate, dehydroacetic acid, sodium dehydroacetate, isobutyl paraoxybenzoate, isopropyl paraoxybenzoate, ethyl paraoxybenzoate, butyl paraoxybenzoate, propyl paraoxybenzoate, benzyl paraoxybenzoate, methyl paraoxybenzoate, sodium paraoxybenzoate methyl, phenoxyethanol, light sensitive dye No. 101, light sensitive dye No. 201 and light sensitive dye No. 401;
antioxidizing agents such as butylhydroxyanisole, butylhydroxytoluene, propyl gallate, erythorbic acid, sodium erythorbate, para-hydroxyanisole and octyl gallate;
chelating agents to bind to a metal ion such as trisodium ethylenediamine hydroxyethyl triacetate, edetic acid, disodium edetate, trisodium edetate, tetrasodium edetate, sodium citrate, gluconic acid, phytic acid, sodium polyphosphate and sodium metaphosphate;
moisturizing agents such as hyaluronic acid, sodium hyaluronate, sodium chondroitin sulfate, sodium lactate, sodium pyrrolidone carboxylate, betaine, lactic acidbacteria fermented solution, yeast extract and ceramide;
anti-inflammatory agents such as glycyrrhizic acid, trisodium glycyrrhizinate, dipotassium glycyrrhizinate, monoammonium glycyrrhizinate, β-glycyrrhetinic acid, glycerin glycyrrhetinate, stearyl glycyrrhetinate, lysozyme chloride, hydrocortisone and allantoin;
pH adjusting agents such as sodium hydroxide, potassium hydroxide and triethanolamine;
salts such as sodium chloride, potassium chloride, magnesium chloride and sodium sulfate;
α-hydroxy acids such as citric acid, glycolic acid, tartaric acid and lactic acid;
whitening agents such as arbutin, α-arbutin and placenta extract;
essential oils such as Archangelica officinalis (angelica) oil, Canangium odoratum (ylang ylang) oil, Canarium luzonicum (elemi) oil, orange oil, Chamomilla recutita (matricaria) oil, Anthemis nobilis oil, Elettaria cardamom (cardamon) oil, Acorus calamus (calamus) oil, Ferula galbaniflua (galbanum) oil, Cinnamomum camphora (camphor) oil, Daucus carota (carrot) seed oil, Salviasclarea (clarysage) oil, Citrusparadisi (grapefruit) oil, Eugenia caryophyllus (clove) oil, Cinnamon bark oil, Coriandrum sativum (coriander) oil, Cupressus sempervirens (cypress) oil, Santalum album (sandalwood) oil, Juniperus virginiana (cedarwood) oil, Cympogon nardus (citronella) oil, Cinnamomum zeylanicum (Cinnamon) leaf oil, Jasmine officinale (jasmine) absolute oil, Juniperus communis (juniper Berry) oil, Zingiber officinale(ginger) extract, Menthaspicata (spearmint) oil, Salvia officinalis (sage) oil, cedar oil, Pelargonium grabeolens (geranium) oil, Thymus vulgaris (thyme) oil, Melaleuca alternifolia (tea tree) oil, Myristica fragrans (nutmeg) oil, Melaleuca qui.viridiflara (niaouli) oil, Citrus aurantium (neroli) oil, pine oil, Ocimum basilicum (basil) oil, Mentha arvensis oil, Pogostemon patchouli (patchouli) oil, Cymbopogon martini (palmarosa) oil, Foeniculumvulgare (fennel) oil, Citrus bigaradia (petitgrain) oil, Piper nigrum (black pepper) oil, Boswellia carterii (frankincense) oil, Vetiveria zizanoides (vetivert) oil, Menthapiperita (peppermint) oil, Citrusbergamia (bergamot) oil, benzoin oil, Aniba rosaeodora (bois de rose) oil, Origanum majorana (marjoram) oil, mandarin oil, Conumiphora myrrha (myrrh) oil, Melissa officinalis (balm mint) oil, Eucalyptus globulus oil, Citrus junos oil, Citrus aurantifolia (lime) oil, Ravensare aromaticum (ravensare) oil, Lavandula latifolia (lavandin) oil, Lavandula angustifolia (lavender) oil, Tilia vulgaris (linden) oil, lemon oil, lemon grass oil, rose oil, Aniba rosaeodora (rosewood) oil, Rosemarinus officinalis (rosemary) oil and Levisticum officinale (lovage) oil;
terpenes such as limonene, pinene, terpinene, terpinolene, myrcene and longifeelene;
fragrance, water, and the like.

Furthermore, to the cosmetic of the invention may also be added any existing raw material of cosmetics at a general concentration. All raw materials of cosmetics described in, for example, Keshouhin genryou kizyun dai-2han chukai (Glossary for Standards of Cosmetic Ingredients, 2nd edition) edited by Society of Japanese Pharmacopoeia, 1984 (YAKUJI NIPPO LIMITED.); Keshouhin genryou kizyun-gai seibun kikaku (The Japanese Cosmetic Ingredients Codex), under the editorship of Pharmaceutical Affairs Bureau Evaluation and Registration Division, 1993 (YAKUJI NIPPO LIMITED.), Keshouhin genryou kizyun-gai seibun kikaku tsuiho (Supplement to The Japanese Cosmetic Ingredients Codex), under the editorship of Pharmaceutical Affairs Bureau Evaluation and Registration Division, 1993 (YAKUJI NIPPO LIMITED.), Keshouhin syubetsu kyoka kizyun (The Comprehensive Licensing Standards of Cosmetics by Category) , under the editorship of Pharmaceutical Affairs Bureau Evaluation and Registration Division, 1993 (YAKUJI NIPPO LIMITED. ) , Keshouhin syubetsu haigou seibun kikaku (The Japanese Cosmetic Ingredients Codex by Category), under the editorship of Pharmaceutical Affairs Bureau Evaluation and Registration Division, 1997 (YAKUJI NIPPO LIMITED.), Keshouhin genryou jiten (Dictionary of rawmaterials of cosmetics), 1991 (Nikko Chemicals Co., Ltd.) and the like may be used.

The amount of these materials contained in the cosmetic is preferably 0.01 to 80 mass%, more preferably 0.1 to 25 mass%, and still more preferably 0.3 to 10 mass% in total amount of the cosmetic.

The cosmetic of the invention obtained in the method as above causes no irritation on skin, and is extremely excellent as: basic skin care cosmetics such as skin toner, lotion, milky lotion, essence, skin cream, cleansing gel, cleansing cream and pack; makeup cosmetics such as makeup base, foundation, eye shadow, lip color and lip gloss; massaging cosmetics such as massaging gel and massaging cream (cold cream) ; and cosmetics for hair such as hair cream, styling gel and hair wax.

### BEST MODE FOR CARRYING OUT THE INVENTION

The present invention is explained in more detail below by way of Examples, however the invention is by no means limited to these Examples. In Examples demonstrated below, glycerin for use had a concentration of 98 mass% or more. Sodium surfactin forusewasAminofect (registered trademark) manufactured by SHOWA DENKO K.K. "%" indicates percentage by mass.

### Example 1 to 10 and Comparative Example 1 to 4: Cleansing cosmetic

Cleansing cosmetics each having a composition as shown in Table 1 were prepared according to the preparation method described below. Storage stability test was performed using these cosmetics.

For the storage stability test, each sample was placed in a glass bottle. After leaving it to stand at 50°C for one week, the condition and appearance were observed. Samples in which some changes such as separation were observed were evaluated "x" while samples in which no change was found were evaluated as "O". In addition, each cosmetic of Examples was subjected to a test for evaluation with respect to washability.

In testing washability, each sample was applied to the skin, and thereafter, sensation of residual oil after washing the applied sample with tap water was evaluated. Samples which gave little sensation of residual oil were evaluated as "O", samples which gave a little sensation of residual oil were evaluated as "Δ", and samples which gave a substantial sensation of residual oil were evaluated "x".

**Table 1: Composition of cosmetic**

| | Example | | | | | | | | | | Comparative Example | | | |
|---|---|---|---|---|---|---|---|---|---|---|---|---|---|---|
| | 1 | 2 | 3 | 4 | 5 | 6 | 7 | 8 | 9 | 10 | 1 | 2 | 3 | 4 |
| Ingredient (% by mass) | | | | | | | | | | | | | | |
| Sodium surfactin | 1 | 0.7 | 0.7 | 0.9 | 0.9 | 0.9 | 0.2 | 2 | 0.9 | 0.9 | 0.9 | - | 0.05 | 10 |
| Glycerin | 24.5 | 19.1 | 19.1 | 37.4 | 37.4 | 37.4 | 24.5 | 24.5 | 37.4 | 37.4 | 37.4 | 37.4 | 24.5 | 24.5 |
| Polyoxyethylene (20) glyceryl triisostearate | 4.7 | 9.7 | 15.4 | - | - | - | 4.7 | 4.7 | 0.2 | - | - | 1.8 | 4.7 | 4.7 |
| Polyoxyethylene (30) sorbit tetraoleate | - | - | - | 13.2 | 17.7 | 1.7 | - | - | - | 0.2 | - | - | - | - |
| Squalane | - | - | - | 33 | 29.5 | 41.9 | - | - | 42.4 | 42.4 | 43.4 | 42.5 | - | - |
| Tri 2-ethylhexanoate glycerin | 65.4 | - | - | 9.9 | 8.9 | 12.5 | 66.3 | 64.4 | 12.7 | 12.7 | 12.7 | 12.7 | 66.4 | 55.4 |
| Tricaprylate/tricaprate glycerin | - | 67.5 | 61.8 | - | - | - | - | - | - | - | - | - | - | - |
| Water | 4.4 | 3 | 3 | 5.6 | 5.6 | 5.6 | 4.4 | 4.4 | 5.6 | 5.6 | 5.6 | 5.6 | 4.4 | 4.4 |
| Results | | | | | | | | | | | | | | |
| Storage stability | ○ | ○ | ○ | ○ | ○ | ○ | ○ | ○ | ○ | ○ | ○ | × | × | × |
| washability | ○ | ○ | ○ | ○ | ○ | ○ | ○ | ○ | Δ | Δ | × | ○ | ○ | ○ |

### Preparation method of cosmetic:

Sodium surfactin was dissolved in glycerin, and thereto were added remaining ingredients except for water little by little while stirring to give a homogenous state. Water was further added thereto, and the mixture was stirred to give a homogenous state.

### Results:

As is clear from Table 1, the cleansing cosmetics of the invention (Examples 1 to 10) exhibited more excellent storage stability or washsability, in comparison with the cleansing cosmetics of Comparative Examples 1 to 4.

### Examples 11 to 13: Cream

According to the method of preparation described below with a composition described in Table 2, creams were prepared.

**Table 2: Composition of cream**

| | Example 11 | Example 12 | Example 13 |
|---|---|---|---|
| Ingredient A (% by mass) | | | |
| Squalane | 8 | 8 | 8 |
| Olive oil | 3 | 3 | - |
| Jojoba oil | 1 | - | 3 |
| Methyl polysiloxane | - | 1 | 1 |
| Cetostearyl alcohol | 1 | 1 | - |
| DL-α-tocopherol | 0.1 | 0.1 | 0.1 |
| Polyoxyethylene (10) glyceryl isostearate | 0.4 | - | 0.4 |
| Polyoxyethylene (40) sorbit tetraoleate | - | 0.4 | - |
| Ingredient B (% by mass) | | | |
| Glycerin | 6 | 6 | 6 |
| 1,3-Butanediol | 1 | 1 | 1 |
| Sodium surfactin | 1 | 1 | 1 |
| Phenoxy ethanol | 0.3 | 0.3 | 0.3 |
| Methylparaben | 0.2 | 0.2 | 0.2 |
| Carboxyvinyl polymer | 0.4 | 0.4 | 0.4 |
| Potassium hydroxide | 0.2 | 0.2 | 0.2 |
| Xanthan gum | 0.2 | 0.2 | 0.2 |
| Fragrance | 0.02 | 0.02 | 0.02 |
| Purified water | balance | balance | balance |

### Method of preparation:

After dissolving ingredients A and ingredients B at 80°C, respectively, they were mixed, and cooled to 30°C while stirring. The mixture was then left to stand to room temperature.

The aforementioned storage stability test was performed using the compositions of Examples 11 to 13. Consequently, excellent stability following storage was exhibited. Thus resulting cream was excellent in moisturizing ability, providing no irritation, with a smooth feeling of use.

### INDUSTRIAL APPLICABILITY

The cosmetic of the invention, with an extremely low skin irritating property, ensures high skin comfort and storage stability. In particular, when used as a cleansing cosmetic, it also exhibits an excellent washability.

## Claims

1. A cleansing cosmetic composition, comprising 0.1 to 5 mass% of a lipopeptide compound, and 0.1 to 20 mass% of a polyoxyethylene glyceryl ether fatty acid ester.

2. The cleansing cosmetic composition as claimed in claim 1, wherein the lipopeptide compound is derived from a microorganism.

3. The cleansing cosmetic composition as claimed in claim 2, wherein the lipopeptide compound is a surfactin represented by the following formula (I), an analogous compound or a salt thereof: 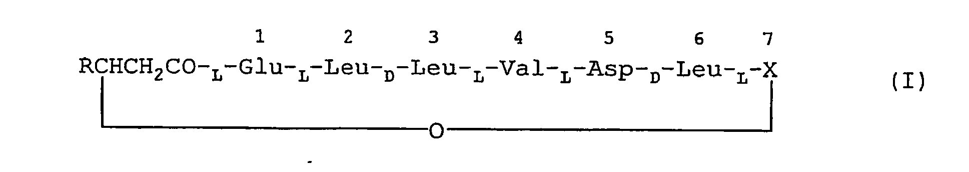 wherein X represents an amino acid residue selected from the group consisting of leucine, isoleucine, valine, glycine, serine, alanine, threonine, asparagine, glutamine, aspartic acid, glutamic acid, lysine, arginine, cysteine, methionine, phenylalanine, tyrosine, tryptophan, histidine, proline, 4-hydroxyproline and homoserine, and R is a normalalkyl group having 8 to 14 carbon atoms, an isoalkyl group having 8 to 14 carbon atoms or an anteiso-alkyl group having 8 to 14 carbon atoms.

4. The cleansing cosmetic composition as claimed in claim 3, wherein the lipopeptide compound is sodium surfactin.

5. The cleansing cosmetic composition as claimed in claim 3, wherein the lipopeptide compound is a compound where the second, fourth and sixth amino acids in the formula (I) are each independently substituted by amino acid selected from a group consisting of leucine, isoleucine, valine, glycine, serine, alanine, threonine, asparagine, glutamine, aspartic acid, glutamic acid, lysine, arginine, cysteine, methionine, phenylalanine, tyrosine, tryptophan, histidine, proline, 4-hydroxyproline and homoserine.

6. The cleansing cosmetic composition as claimed in any one of claims 1 to 5, wherein the cosmetic is in gel form or cream form.

7. The use of a cosmetic composition, comprising 0.1 to 5 mass % of a lipopeptide compound and 0.1 to 20 mass% of a polyoxyethylene glyceryl ether fatty acid ester as a cleansing cosmetic.

## Patentansprüche

1. Kosmetische Reinigungszusammensetzung, welche 0,1 bis 5 Massen-% einer Lipopeptidverbindung und 0,1 bis 20 Massen-% eines Polyoxyethylen-Glycerylether-Fettsäureesters enthält.

2. Kosmetische Reinigungszusammensetzung nach Anspruch 1, wobei die Lipopeptidverbindung von einem Mikroorganismus abgeleitet ist.

3. Kosmetische Reinigungszusammensetzung nach Anspruch 2, wobei die Lipopeptidverbindung ein Surfactin der folgenden Formel (1), eine analoge Verbindung oder ein Salz davon ist: 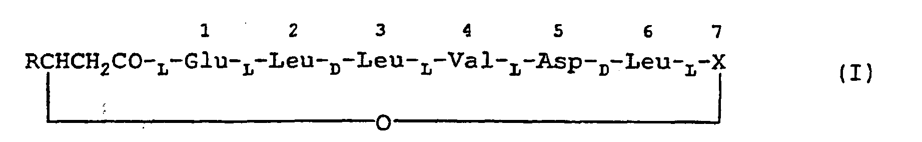 worin X einen Aminosäurerest darstellt, der aus der aus Leucin, Isoleucin, Valin, Glycin, Serin, Alanin, Threonin, Asparagin, Glutamin, Asparaginsäure, Glutaminsäure, Lysin, Arginin, Cystein, Methionin, Phenylalanin, Tyrosin, Tryptophan, Histidin, Prolin, 4-Hydroxyprolin und Homoserin bestehenden Gruppe ausgewählt ist, und R eine Normalalkylgruppe mit 8 bis 14 Kohlenstoffatomen, eine Isoalkylgruppe mit 8 bis 14 Kohlenstoffatomen, und eine Anteisoalkylgruppe mit 8 bis 14 Kohlenstoffatomen ist.

4. Kosmetische Reinigungszusammensetzung nach Anspruch 3, wobei die Lipopeptidverbindung Natriumsurfactin ist.

5. Kosmetische Reinigungszusammensetzung nach Anspruch 3, wobei die Lipopeptidverbindung eine Verbindung ist, in der die zweite, vierte und sechste Aminosäure in der Formel (I) jeweils unabhängig voneinander durch eine Aminosäure substituiert sind, die aus der aus Leucin, Isoleucin, Valin, Glycin, Serin, Alanin, Threonin, Asparagin, Glutamin, Asparaginsäure, Glutaminsäure, Lysin, Arginin, Cystein, Methionin, Phenylalanin, Tyrosin, Tryptophan, Histidin, Prolin, 4-Hydroxyprolin und Homoserin bestehenden Gruppe ausgewählt ist.

6. Kosmetische Reinigungszusammensetzung nach einem der Ansprüche 1 bis 5, wobei das Kosmetikum Gelform oder Cremeform hat.

7. Verwendung einer kosmetischen Zusammensetzung, die 0,1 bis 5 Massen-% einer Lipopeptidverbindung und 0,1 bis 20 Massen-% eines Polyoxyethylen-Glycerylether-Fettsäureesters enthält, als Reinigungskosmetikum.

## Revendications

1. Composition de cosmétique nettoyant comprenant 0,1 à 5 % en masse d'un composé lipopeptidique et 0,1 à 20 % en masse d'un ester d'acide gras et de polyoxyéthylèneglycéryléther.

2. Composition de cosmétique nettoyant selon la revendication 1 où le composé lipopeptidique est dérivé d'un microorganisme.

3. Composition de cosmétique nettoyant selon la revendication 2 où le composé lipopeptidique est une surfactine représentée par la formule (I) suivante, un composé analogue ou un sel de celle-ci : 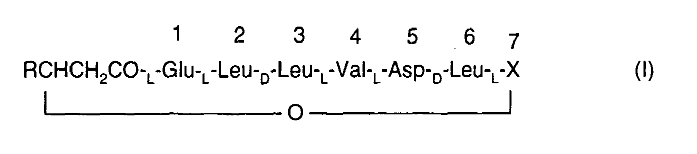 où X représente un résidu d'aminoacide choisi dans le groupe consistant en la leucine, l'isoleucine, la valine, la glycine, la sérine, l'alanine, la thréonine, l'asparagine, la glutamine, l'acide aspartique, l'acide glutamique, la lysine, l'arginine, la cystéine, la méthionine, la phénylalanine, la tyrosine, le tryptophane, l'histidine, la proline, la 4-hydroxyproline et l'homosérine et R est un groupe alkyle normal ayant 8 à 14 atomes de carbone, un groupe isoalkyle ayant 8 à 14 atomes de carbone ou un groupe antéiso-alkyle ayant 8 à 14 atomes de carbone.

4. Composition de cosmétique nettoyant selon la revendication 3 où le composé lipopeptidique est une surfactine sodique.

5. Composition de cosmétique nettoyant selon la revendication 3 où le composé lipopeptidique est un composé où les second, quatrième et sixième aminoacides dans la formule (I) sont substitués chacun indépendamment par un aminoacide choisi dans un groupe consistant en la leucine, l'isoleucine, la valine, la glycine, la sérine, l'alanine, la thréonine, l'asparagine, la glutamine, l'acide aspartique, l'acide glutamique, la lysine, l'arginine, la cystéine, la méthionine, la phénylalanine, la tyrosine, le tryptophane, l'histidine, la proline, la 4-hydroxyproline et l'homosérine.

6. Composition de cosmétique nettoyant selon l'une quelconque des revendications 1 à 5 où le cosmétique est sous forme de gel ou sous forme de crème.

7. Utilisation d'une composition de cosmétique comprenant 0,1 à 5 % en masse d'un composé lipopeptidique et 0,1 à 20 % en masse d'un ester d'acide gras et de polyoxyéthylèneglycéryléther comme cosmétique nettoyant.
